# EUROPEAN PATENT SPECIFICATION

(11) **EP 3 005 981 B1**
(45) Date of publication and mention of the grant of the patent: **04.07.2018**
(21) Application number: 14814415.7
(22) Date of filing: 09.06.2014
(51) Int. Cl.: A61F 2/16, B29D 11/02, A61F 2/14

(54) **IMPLANTABLE MYOPIA LENS AND PREPARATION METHOD THEREFOR**
IMPLANTIERBARE LINSEN ZUR KORREKTUR VON KURZSICHTIGKEIT UND HERSTELLUNGSVERFAHREN DAFÜR
LENTILLE IMPLANTABLE POUR MYOPIE ET PROCÉDÉ DE PRÉPARATION DE LADITE LENTILLE

(30) Priority: 17.06.2013 CN 201310240602
(43) Date of publication of application: 13.04.2016
(73) Proprietor: Wuxi Vision Pro Ltd., Wuxi, Jiangsu 214125 (CN)
(72) Inventor: LIAO, Xiugao, Wuxi Jiangsu 214125 (CN)
(74) Representative: Sun, Yiming
(86) International application number: PCT/CN2014/079477
(87) International publication number: WO 2014/201956

(56) References cited:
- CN-A- 101 021 622
- CN-A- 101 039 635
- CN-A- 101 050 294
- CN-A- 101 050 294
- CN-A- 103 340 703
- CN-A- 103 705 973
- CN-U- 203 341 853
- US-A1- 2003 097 177
- US-A1- 2003 187 505
- US-A1- 2007 239 274
- US-A1- 2012 296 425

## Description

### Technical Field

The invention relates to the technical field of production and fabrication of implantable Myopia Lens, in particular to an implantable myopia lens and a preparation method thereof.

### Background

US 2007/0239274 A1 describes an intraocular lens assembly or method for correcting myopia, hyperopia and astigmatism using the intraocular lens assembly. The intraocular lens assembly relates to intraocular lenses which provide accommodation. The intraocular lens assembly comprises a lens extending along an optical axis between an anterior optical surface and a posterior optical surface. The intraocular lens has a circumferential edge disposed about the optical axis at a junction of anterior and posterior optical surfaces. The intraocular lens assembly further has at least two haptics. Each haptic extends from an associated portion of the circumferential edge and along an associated haptic axis. In addition, each haptic is loop-like or paddle-like and extends between end portions at opposite ends thereof. The end portions are joined to the lens at the circumferential edge. Each of the haptics includes at least one footplate extending symmetrically about is associated haptic axis. The resultant vaulted structure provides an intraocular lens assembly that, when implanted in the eye, allows accommodation.

Eyes of human are colorless and transparent at birth. Intraocular lenses are soft, colorless and transparent, and can effectively focus at relaxation and tension to make human see very clearly at a far and near distance. But as newborn infants grow up, some will get nearsighted. Especially with the development in computer, television and network, young students spend a lot of time on computers and Internet. Frequent watching a display at a short distance harms eyes greatly, making more and more people nearsighted, and severer. Some have a myopia up to one thousand degrees and have difficulties in walking and seeing without help of glasses. But, when eyeglasses are used, the lenses often develops vapor whether having a meal or noodles, and the lenses may get broken if not used correctly. People with a high degree of myopia must wear myopia lenses with high degree, and the lenses look like circles, which directly affects the appearance of the user. Although wearing contact lenses looks very natural, the contact lenses need to be removed and washed regularly. Meanwhile, the contact lenses require material with excellent air permeability, and must be changed for new ones after a period of time, which is very inconvenient. In order to eliminate the troubles with wearing glasses, since the 1990s, people have begun to use laser technique to treat the shape of corneas, so as to eliminate myopia. But treating the corneas by the laser technique would often lead to dry eyes, and more seriously lead to cornea detachment and blindness for the patient.

With the context of the disadvantages of the above techniques, people have begun the design and material development for the implantable myopia lens. In U.S. patent No. 5,258,025, 5,480,428 and 5,766,245, Fedorov S et. al. first proposed the design of the implantable myopia lens and specific part where the implantable myopia lens is implanted into an eye. In U.S. patent No. 5,913,898, Feingold V first proposed an implantable contact lens (ICL). In U.S. patent No. 6,015,435, 6,428,574, 6,506,212 and 6,706,066, Valunin Igor et. al. first proposed the design of posterior chamber floating implantable myopia lens.

Materials disclosed in the prior art for developing the implantable myopia lens mainly include PMMA material, silica gel material and hydrophobic polyacrylate material. The PMMA material is too rigid to fold, which needs to open a large side incision during implanting operation, resulting in too many cells loss and great harms to ocular tissues. Both the silica gel material and the hydrophobic polyacrylate material are hydrophobic materials, also very rigid and easy to cause induced cataract after being implanted into the posterior chamber.

### Summary of the Invention

In order to solve the technical problems described above, the invention provides an implantable myopia lens and a preparation method thereof, to achieve the objectives of enabling the lens to automatically adjusting in size according to the size of human eyes, enhancing use comfortability and facilitating convenient implanting into human eyes.

In order to achieve above objectives, the technical schemes of the invention are as follows:
an implantable myopia lens, wherein, said lens is to be located in a posterior chamber of a human eye, said lens comprising: a lens body and a plurality of sheet-shaped support arms, cut and formed along an edge of said lens body; wherein each of said support arms includes a connection portion and a support portion; wherein said connection portion is connected with the edge of said lens body; wherein an angle between a plane of said support arm and a plane of said lens body is between 10 and 20 degrees, making the whole of the lens a spheroid, wherein a transparent light guide zone is arranged on said lens body, wherein a bending angle of said support arms automatically adjusts to a contour of said human eye, when said lens is implanted therein, and said lens body and said support arms are all made of a same hydrophilic polyacrylate material.

Preferably, said connection portion is a thin and short structure.

Preferably, a plurality of through holes are arranged on said lens body, and said through holes are not in said transparent light guide zone.

Preferably, said hydrophilic polyacrylate material is made from copolymerization of 2-hydroxyethyl methacrylate and alkyl acrylate derivatives.

Preferably, said hydrophilic polyacrylate material is made from copolymerization of alkyl acrylate, alkyl methacrylate and styrene or styrene derivatives.

Preferably, said hydrophilic polyacrylate material also contains ultraviolet absorbent and blue light absorbent.

A preparation method of said implantable myopia lens comprises the following steps:
a. Aerating nitrogen into small molecule monomer mixture solution of hydrophilic acrylates to remove air dissolved in the solution, after 60 minutes, transfer this solution to a forming mould under nitrogen atmosphere, form sheet-shaped hydrophilic polyacrylate material with a thickness of about 3 mm at high temperature, and then cut the sheet-shaped material described above into circular rough lenses;
b. Placing the cut circular rough lenses into an extraction device, extract with alcohol solvent to remove unpolymerized small molecules and medium-sized molecules, and dry subsequently; and
c. Cutting the rough lenses to prepare the lens body with the transparent light guide zone, polish to remove tool marks, and finally cut out the support arms to obtain the lens.

Through the technical schemes described above, the support arms of the implantable myopia lens provided by the invention are bendable, and the bending angle of the support arms is automatically adjusted according to the contour of a human eye when the lens is implanted therein. The lens of the same model can be implanted into human eyes of different sizes. The lens is better fit to human eyes and more comfortable because the connections between the support arms and the lens body are thin and short, and the support arms bend more easily. The lens material has a refractive index of 1.430-1.490 and low thickness, the hydrophilic polyacrylate material is highly elastic, has a recovery time less than 1 second within human eyes, and needs a small side incision to be opened during implanting operation, making the lens easy to be implanted into the human eye.

### Brief Description of the Drawings

In order to better illustrate the Examples of the invention or technical schemes in the prior art, figures required in description of the Examples or the prior art will be simply introduced below.
Fig. 1 is a front-view schematic diagram of an implantable myopia lens disclosed in Example 1 of the invention;
Fig. 2 is a side-view schematic diagram of an implantable myopia lens disclosed in Example 1 of the invention;
Fig. 3 is a front-view schematic diagram of an implantable myopia lens disclosed in Example 2 of the invention; and
Fig. 4 is a side-view schematic diagram of an implantable myopia lens disclosed in Example 2 of the invention.

### Detailed Description of the Preferred Embodiments

The technical schemes in Examples of the invention will be described clearly and fully in conjunction with drawings in Examples of the invention.

### Example 1

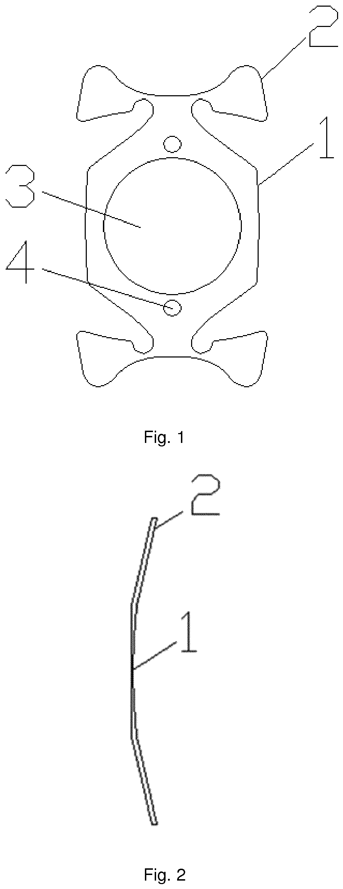

According to Figs. 1-2, the invention discloses an implantable myopia lens, comprising a lens body 1 and four sheet-shaped support arms 2 cut and formed along the edge of the lens body 1. The support arm 2 includes a thin and short connection portion and a flat support portion, wherein, the connection portion is connected with the edge of the lens body 1, the angle between the plane of the support arms 2 and the plane of the lens body 1 is between 10 and 20 degrees, making the whole of the lens a spheroid; a transparent light guide zone 3 is arranged on the lens body 1, the transparent light guide zone 3 is a concave lens with a diameter of 4.5-6.0 mm and a central thickness of 0.05-0.25 mm, and the lens body 1 and the support arms 2 are all made of the same hydrophilic polyacrylate material.

In order to facilitate exchange of human body fluid on both sides of the lens, two circular through holes 4 are arranged on the lens body 1, and the through holes 4 are not in the transparent light guide zone 3.

### Example 2

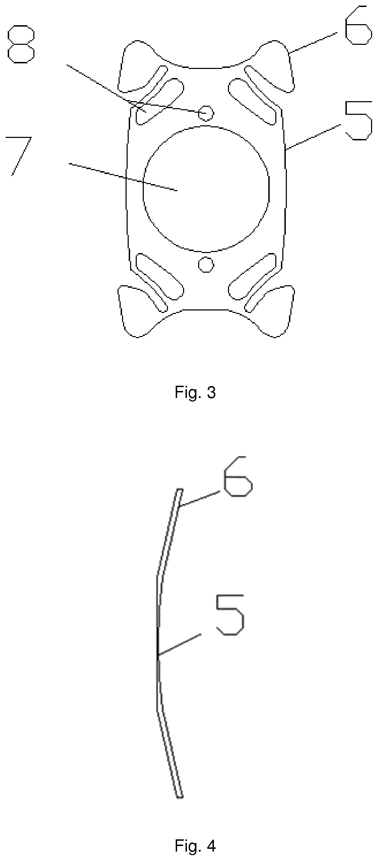

According to Figs. 3-4, the invention discloses an implantable myopia lens, comprising a lens body 5 and four sheet-shaped support arms 6 cut and formed along the edge of the lens body 5, wherein, the support arm 6 includes a thin and short connection portion and a flat support portion, the connection portion is connected with the edge of the lens body 5, the distance between the inner edges of the support arms 6 and the edge of the lens body 5 is shorter than that between the inner edges of the support arms 2 and the edge of the lens body 1 in Example 1, and the oscillating range of the support arms 6 is smaller after being implanted into human eyes. The angle between the plane of the support arms 6 and the plane of the lens body 5 is between 10 and 20 degrees, making the whole of the lens a spheroid, a transparent light guide zone 7 is arranged on the lens body 5, the transparent light guide zone 7 is a concave lens with a diameter of 4.5-6.0 mm and a central thickness of 0.05-0.25 mm, and the lens body 5 and the support arms 6 are all made of the same hydrophilic polyacrylate material.

In order to facilitate exchange of human body fluid on both sides of the lens, two circular through holes 8 and four strip-shaped through holes 8 are arranged on the lens body 5, compared with Example 1, the four strip-shaped through holes added make exchange of human body fluid on both sides of the lens more smooth, and the through holes 8 are not in the transparent light guide zone 7.

The hydrophilic polyacrylate material used in the invention is made from copolymerization of 2-hydroxyethyl methacrylate and alkyl acrylate derivatives, or made from copolymerization of alkyl acrylate, alkyl methacrylate and styrene or styrene derivatives.

Wherein, said hydrophilic polyacrylate material can also contain polymerizable medium crosslinkable molecules or crosslinkable groups; the polymerizable crosslinkable molecules include: ethylene glycol dimethacrylate diester, propylene glycol dimethacrylate diester, butanediol dimethacrylate diester, hexanediol dimethacrylate diester, ethylene glycol diacrylate diester, propylene glycol diacrylate diester, butanediol diacrylate diester, hexanediol diacrylate diester, 1,4-divinyl benzene or 1,3-divinyl benzene; the crosslinkable groups are: methacryloyl group, acryloyl group or vinyl phenyl group.

The hydrophilic polyacrylate material also contains ultraviolet absorbent and blue light absorbent, and the ultraviolet absorbent also contains benzophenone or benzotriazole group.

The implantable myopia lens provided by the invention is located in the posterior chamber in an eye after being implanted, with the support of the support arms, the transparent light guide zone is located at a central pupilla within the eye, the outer surface of the transparent light guide zone can be spherical, aspherical or Toric optical surface, and the hydrophilic polyacrylate material has a refractive index ranging from 1.450 to 1.490, therefore the lens has a smaller thickness, not easy to contact the capsular bag to cause induced cataract.

The preparation method of the implantable myopia lens disclosed in the invention is as follows:
a. Placing small molecule monomer mixture solution of hydrophilic acrylates monomers prepared in advance in a 3L three-necked bottle, aerate nitrogen to remove air dissolved in the solution, after about 60 minutes, transfer the small molecule monomer mixture solution to a forming mould under nitrogen atmosphere, form sheet-shaped hydrophilic polyacrylate material with a thickness of about 3 mm, a length of 10.5 inches and a width of 9.5 inches at high temperature, and then cut the sheet-shaped material described above into circular rough lenses;
b. Placing the cut circular rough lenses into an extraction device, extract with alcohol solvent to remove unpolymerized small molecules and medium-sized molecules, and dry subsequently; and
c. Cutting the rough lenses by DAC or Optoform cutter to prepare the lens body with the transparent light guide zone, polish to remove tool marks, and finally cut out the support arms to obtain the lens.

The support arms of the implantable myopia lens provided by the invention are bendable, and the bending angle of the support arms is automatically adjusted according to the contour of a human eye when the lens is implanted therein. The lens of the same model can be implanted into human eyes of different sizes. The lens is better fit to human eyes and more comfortable because the connections between the support arms and the lens body are thin and short, and the support arms bend more easily. The lens material has a refractive index of 1.430-1.490 and low thickness, the hydrophilic polyacrylate material is highly elastic, has a recovery time less than 1 second within human eyes, and needs a small side incision to be opened during implanting operation, making the lens easy to be implanted into the human eye.

The above description of the disclosed Examples enables those skilled in the art to realize or use the invention. It is obvious for a person skilled in the art to have modifications based on the embodiments, and the general principles defined herein could be realized in other Examples without departing from the scope of the invention.

## Claims

1. An implantable myopia lens, wherein said lens is to be located in a posterior chamber of a human eye, said lens comprising:
a lens body (1, 5), and
a plurality of sheet-shaped support arms (2, 6), cut and formed along an edge of said lens body; wherein each of said support arms includes a connection portion (21, 61) and a support portion; wherein said connection portion is connected with the edge of said lens body; wherein an angle between a plane of said support arm and a plane of said lens body is between 10 and 20 degrees, making the whole of the lens a spheroid, wherein a transparent light guide zone (3, 7) is arranged on said lens body; wherein a bending angle of said support arms automatically adjusts to a contour of said human eye, when said lens is implanted therein,
**characterized in that**
said lens body and said support arms are all made of a same hydrophilic polyacrylate material.

2. The implantable myopia lens according to claim 1, wherein said connection portion is a thin and short structure.

3. The implantable myopia lens according to claim 1, wherein a plurality of through holes (4, 8) is arranged on said lens body, and said through holes are not in said transparent light guide zone.

4. The implantable myopia lens according to claim 1, wherein said hydrophilic polyacrylate material is made from copolymerization of 2-hydroxyethyl methacrylate and alkyl acrylate derivatives.

5. The implantable myopia lens according to claim 1, wherein said hydrophilic polyacrylate material is made from copolymerization of alkyl acrylate, alkyl methacrylate and styrene.

6. The implantable myopia lens according to Claim 1 or 4 or 5, wherein said hydrophilic polyacrylate material also contains ultraviolet absorbent and blue light absorbent.

7. A preparation method of an implantable lens, wherein the method comprises the following steps:
a. Aerating nitrogen into small molecule monomer mixture solution of hydrophilic acrylic monomers to remove air dissolved in the solution, after 60 minutes, transfer this solution to a forming mould under nitrogen atmosphere, form sheet-shaped polyacrylate material with a thickness of about 3 mm at high temperature, and then cut the sheet-shaped material described above into circular rough lenses;
b. Placing the cut circular rough lenses into an extraction device, extract with alcohol solvent to remove unpolymerized small molecules and medium-sized molecules, and dry subsequently; and
c. Cutting the rough lenses to prepare the lens body (1, 5) with the transparent light guide zone (3, 7), polish to remove tool marks, and finally cut out the support arms (2, 6) to obtain the lens;
**characterized in that**
the implantable lens is an implantable myopia lens according to Claim 1.

## Patentansprüche

1. Implantierbare Myopie-Linse, wobei die Linse in einer Hinterkammer eines menschlichen Auges angeordnet ist, wobei die Linse umfasst:
ein Linsenkörper (1, 5), und
eine Vielzahl von blattförmigen Tragarmen (2, 6), die entlang einer Kante des Linsenkörpers ausgeschnitten und geformt sind; wobei jeder der Tragarme einen Verbindungsabschnitt (21, 61) und einen Stützabschnitt aufweist; wobei der Verbindungsabschnitt mit der Kante des Linsenkörpers verbunden ist; wobei ein Winkel zwischen einer Ebene des Tragarms und einer Ebene des Linsenkörpers zwischen 10 und 20 Grad beträgt, wodurch die gesamte Linse sphäroidisch wird, wobei eine transparente Lichtleitzone (3, 7) an dem Linsenkörper angeordnet ist; wobei sich ein Biegewinkel der Tragarme automatisch an eine Kontur des menschlichen Auges anpasst, wenn die Linse darin implantiert ist,
**dadurch gekennzeichnet, dass**
der Linsenkörper und die Tragarme alle aus demselben hydrophilen Polyacrylatmaterial hergestellt sind.

2. Implantierbare Myopie-Linse nach Anspruch 1, wobei der Verbindungsabschnitt eine dünne und kurze Struktur ist.

3. Implantierbare Myopie-Linse nach Anspruch 1, wobei eine Vielzahl von Durchgangslöchern (4, 8) auf dem Linsenkörper angeordnet ist und die Durchgangslöcher nicht in der transparenten Lichtleitzone liegen.

4. Implantierbare Myopie-Linse nach Anspruch 1, wobei das hydrophile Polyacrylatmaterial aus der Copolymerisation von 2-Hydroxyethylmethacrylat und Alkylacrylatderivaten hergestellt ist.

5. Implantierbare Myopie-Linse nach Anspruch 1, wobei das hydrophile Polyacrylatmaterial aus einer Copolymerisation von Alkylacrylat, Alkylmethacrylat und Styrol hergestellt ist.

6. Implantierbare Myopie-Linse nach Anspruch 1 oder 4 oder 5, wobei das hydrophile Polyacrylatmaterial ebenfalls Ultraviolettabsorbtionsmittel und Blaulichtabsorbtionsmittel enthält.

7. Verfahren zur Herstellung einer implantierbaren Linse, wobei das Verfahren die folgenden Schritte umfasst:
a. Durchlüften von Stickstoff in eine Lösung von niedermolekularer Monomermischung von hydrophilen Acrylmonomeren, um in der Lösung gelöste Luft zu entfernen, überführt diese Lösung nach 60 Minuten in eine Formgebungsform unter Stickstoffatmosphäre, um blattförmiges Polyacrylatmaterial mit einer Dicke von etwa 3 mm unter hoher Temperatur zu bilden und dann Schneiden des oben beschriebenen blattförmigen Materials in kreisförmige rohe Linsen;
b. Platzieren der geschnittenen kreisförmigen Rohlinsen in eine Extraktionsvorrichtung, Extrahieren mit Alkohollösungsmittel, um nichtpolymerisierte kleine Moleküle und mittelgroße Moleküle zu entfernen und anschließend zu trocknen; und
c. Schneiden der Rohlinsen, um den Linsenkörper (1, 5) mit der transparenten Lichtleitzone (3, 7) vorzubereiten, polieren, um Werkzeugmarkierungen zu entfernen, und schließlich die Tragarme (2, 6) ausschneiden, um die Linse zu erhalten;
**dadurch gekennzeichnet, dass**
die implantierbare Linse eine implantierbare Myopie-Linse gemäß Anspruch 1 ist.

## Revendications

1. Lentille pour myopie implantable, dans laquelle ladite lentille doit se situer dans une chambre postérieure d'un oeil humain, ladite lentille comprenant :
un corps de lentille (1, 5), et
une multitude de bras de soutien en forme de feuilles (2, 6), découpés et formés le long d'un bord dudit corps de lentille; dans laquelle chacun desdits bras de soutien comprend une partie de raccordement (21, 61) et une partie de soutien ; dans laquelle ladite partie de raccordement est raccordée au bord dudit corps de lentille ; dans laquelle un angle entre un plan dudit bras de soutien et un plan dudit corps de lentille est compris entre 10 et 20 degrés, ce qui fait que la totalité de la lentille constitue un sphéroïde, dans laquelle une zone de guidage de lumière transparente (3, 7) est disposée sur ledit corps de lentille ; dans laquelle un angle de pliage desdits bras de soutien s'ajuste automatiquement à un contour dudit oeil humain, lorsque ladite lentille y est implantée,
**caractérisé en ce que**
ledit corps de lentille et lesdits bras de soutien sont tous fabriqués en un même matériau de polyacrylate hydrophile.

2. Lentille pour myopie implantable selon la revendication 1, dans laquelle ladite partie de raccordement est une structure fine et courte.

3. Lentille pour myopie implantable selon la revendication 1, dans laquelle une pluralité de trous traversants (4, 8) est disposée sur ledit corps de lentille, et lesdits trous traversants ne sont pas dans ladite zone de guidage de lumière transparente.

4. Lentille pour myopie implantable selon la revendication 1, dans laquelle ledit matériau de polyacrylate hydrophile est formé par copolymérisation de méthacrylate de 2-hydroxyéthyle de dérivés d'acrylate d'alkyle.

5. Lentille pour myopie implantable selon la revendication 1, dans laquelle ledit matériau de polyacrylate est formé par copolymérisation d'acrylate d'alkyle, de méthacrylate d'alkyle et de styrène.

6. Lentille pour myopie implantable selon la revendication 1 ou 4 ou 5, dans laquelle ledit matériau de polyacrylate hydrophile contient également un absorbeur d'ultraviolets et un absorbeur de lumière bleue.

7. Procédé de préparation d'une lentille implantable, dans lequel le procédé comprend les étapes suivantes :
a. aération d'azote dans une solution de mélange monomère à petites molécules de monomères acryliques hydrophiles pour éliminer l'air dissous dans la solution, après 60 minutes, le transfert de cette solution à un moule de formage sous atmosphère d'azote, la formation d'un matériau de polyacrylate en forme de feuille avec une épaisseur d'environ 3 mm à température élevée, puis la découpe du matériau de feuille décrit ci-dessus en lentilles rugueuses circulaires ;
b. le positionnement des lentilles rugueuses circulaires découpées dans un dispositif d'extraction, l'extraction avec un solvant alcoolique pour éliminer les petites molécules non polymérisées et les molécules de taille moyenne, puis le séchage ; et
c. la découpe des lentilles rugueuses pour préparer le corps de lentille (1, 5) avec la zone de guidage de lumière transparente (3, 7), le polissage pour éliminer les marques d'outils, et finalement la découpe des bras de soutien (2, 6) pour obtenir la lentille ;
**caractérisé en ce que**
la lentille implantable est une lentille pour myopie implantable selon la revendication 1.
